# EUROPÄISCHE PATENTSCHRIFT

(11) **EP 3 445 777 B1**
(45) Veröffentlichungstag und Bekanntmachung des Hinweises auf die Patenterteilung: **26.02.2020**
(21) Anmeldenummer: 17723236.0
(22) Anmeldetag: 21.04.2017
(51) Int. Cl.: C07K 14/52, A61K 31/592, A61K 38/19, A61K 31/593

(54) **SELEKTIV DEGLYCOSILIERTES VITAMIN D BINDENDES PROTEIN (GCMAF) UND CHOLECALCIFEROL (CALCIOL) SOWIE HERSTELLUNGSVERFAHREN**
SELECTIVELY DEGLYCOSYLATED VITAMIN D-BINDING PROTEIN (GCMAF), CHOLECALCIFEROL (CALCIOL), AND PRODUCTION METHOD
PROTÉINE DÉGLYCOSYLÉE SÉLECTIVEMENT LIANT LA VITAMINE D (GCMAF), CHOLÉCALCIFÉROL (CALCIOL) ET PROCÉDÉ DE PRÉPARATION

(30) Priorität: 21.04.2016 AT 503542016
(43) Veröffentlichungstag der Anmeldung: 27.02.2019
(73) Patentinhaber: HG Pharma GmbH, 1170 Wien (AT)
(72) Erfinder: HERWIG, Ralf, 6363 Westendorf (AT); GREILBERGER, Joachim, 8111 Judendorf-Strassengel (AT)
(74) Vertreter: Müllner, Martin
(86) Internationale Anmeldenummer: PCT/AT2017/060104
(87) Internationale Veröffentlichungsnummer: WO 2017/181213

(56) Entgegenhaltungen:
- WO-A1-01/85194
- WO-A1-2012/137199
- WO-A1-2014/202956

## Beschreibung

### Technisches Gebiet

Die vorliegende Erfindung betrifft selektiv deglycosiliertes Vitamin D bindenedes Protein (GcMAF) und Cholecalciferol (Calciol) sowie ein Herstellungsverfahren.

### Stand der Technik

Cholecalciferol, auch Calciol genannt, ist bekanntlich die wichtigste physiologische Form von Vitamin D im Menschen. Es wird daher oft in Nahrungsergänzungsmitteln verwendet. Es wird im Körper zu Calcitriol umgewandelt. Calcitriol unterscheidet sich von Calciol durch zwei OH-Gruppen an den Positionen 1 und 25.

Calciol und auch Calcidiol und Calcitriol sind hydrophob; Vitamin D wird daher im Blut durch das Vitamin D bindende Protein transportiert, das auch als Gc-Globulin (gc=group specific component) bezeichnet wird. Wenn man das Vitamin D bindende Protein selektiv deglykosyliert, erhält man ein Molekül, das ein wirkungsvoller Makrophagen-Aktivator ist. Dies ist z.B. aus Absatz [0009] der patcit0001:WO WO 01/85194 A --.
bekannt. Dieses Molekül wird daher im Folgenden mit GcMAF (MAF=M akrophagen aktivierender Faktor) bezeichnet. Aus der genannten Schrift ist bekannt, dass GcMAF zum Beispiel bei der Bekämpfung von Tumoren wirkungsvoll ist. Es wird angenommen, dass GcMAF die Makrophagen aktiviert, welche dann ihrerseits die Tumorzellen angreifen (Absatz [0010] der genannten Schrift).

Andererseits ist auch bekannt, dass Calcitriol bei der Tumorbekämpfung hilfreich ist, insbesondere bei Prostata-Karzinomen (Absatz [0064] dieser Schrift).

In dieser Schrift wird daher auch vorgeschlagen, eine Zusammensetzung umfassend GcMAF und Calcitriol an Krebspatienten zu verabreichen (Absatz [0016]), insbesondere an Patienten mit Prostatakrebs und Brustkrebs (Absatz [0066]). Mangels Angabe eines konkreten Herstellungsverfahrens ist davon auszugehen, dass die beiden Komponenten nebeneinander vorliegen und nicht chemisch aneinander gebunden sind.

Gemäß
patcit0002:WO WO 2014/202956 A --.
wird ein trimerer Komplex von GcMAF, Calcitriol und einer ungesättigten Fettsäure hergestellt. Es wird zuerst GcMAF mit Calcitriol reagieren gelassen, sodass als Zwischenprodukt ein dimerer Komplex GcMAF-Calcitriol entsteht.

Es wurde nun im Zuge der vorliegenden Erfindung überraschender Weise gefunden, dass ein Komplex GcMAF-Calciol dem Komplex GcMAF-Calcitriol überlegen ist: es ist die Phagocytose-Aktivität höher und die Bildung von Superoxid-Radikal-Anionen (O₂^{•-}) geringer.

### Darstellung der Erfindung

Die Erfindung betrifft daher einen dimeren Komplex aus selektiv deglycosyliertem Vitamin D bindenden Protein (GcMAF) und Cholecalciferol (Calciol), insbesondere als Arzneimittel.

Wie im Rahmen der vorliegenden Erfindung weiters festgestellt wurde, hängt die Wirksamkeit signifikant von der Qualität der Reinigung ab. Die Erfindung betrifft daher auch ein Verfahren zur Herstellung solch eines Komplexes, indem man Vitamin D bindendes Protein mit Calciol reagieren lässt und davor oder danach selektiv deglycosyliert, wobei man schließlich das sich ergebende Produkt mit einem Molekularsieb mit einem Cut-Off-Wert von mehr als 60 kDa von Verunreinigungen größeren Molekulargewichts, insbesondere von Enzymen der selektiven Deglycosylierung, trennt, vorzugsweise auch mit einem Molekularsieb mit einem Cut-Off-Wert von höchstens 10 kDa von abgespaltenen Zuckerresten trennt.

Nach den im Rahmen der vorliegenden Erfindung durchgeführten Messungen ist die Phagozytose-Aktivität des bekannten Komplexes GcMAF-Calcitriol praktisch identisch mit der Phagocytose-Aktivität von GcMAF allein; der Komplex GcMAF-Calciol hat hingegen eine signifikant höhere Phagozytose-Aktivität.

Eine geringere Bildung von Superoxid-Radikal-Anionen ist ein Vorteil: zwar schaden Superoxid-Radikal-Anionen den Tumorzellen, aber in ähnlichem Ausmaß auch den gesunden Zellen und den Macrophagen, die dann bei der Tumorbekämpfung nur noch eine geringere Wirkung entfalten können, was die direkte Wirkung der Superoxid-Radikal-Anionen mehr als zunichtemacht.

Der Standard-Test auf Vitamin D im Blut, ein ELISA-Test, ist selektiv auf Calcidiol (das an der Position 25 eine OH-Gruppe hat). Bei diesem Test ist es notwendig, zunächst das Calcidiol vom Vitamin D bindenden Protein zu trennen, denn der Komplex Gc-Calcidiol bindet nicht an die Rezeptorstellen des ELISA-Tests. Da die Rezeptorstellen des ELISA-Tests ähnlich den Vitamin D-Rezeptoren des menschlichen Körpers sind, kann man daraus schließen, dass dieser Komplex auch nicht an diese Vitamin D-Rezeptoren bindet. Das gleiche gilt für den Komplex GcMAF-Calcitriol. Überraschender Weise hat sich jedoch gezeigt, dass der Komplex GcMAF-Calciol sehr wohl an die Rezeptorstellen des ELISA-Tests bindet und somit auch an die Vitamin D-Rezeptoren des menschlichen Körpers binden wird; dies ist wahrscheinlich ein Grund für die überlegene Wirkung.

### Weg(e) zur Ausführung der Erfindung

Anhand der folgenden Beschreibung wird die Erfindung näher erläutert.

Es wird zunächst das Herstellungsverfahren beschrieben: Der erfindungsgemäße Komplex wird aus Gc-Protein oder rekombinantem Gc-Protein mit anschließender Cholecalciferol-Bindung hergestellt.

### Herstellung der immobilisierten Enzyme mittels Sepharose 4B oder Cyano-aktivierten Magnetic beads oder anderen Spaltungsmöglichkeiten

Verwendete Puffer bzw. Lösungen:
Coupling Puffer: 16,8 g NaHCO₃ und 58,44 g NaCl in 21 Wasser lösen
0,2 M Glycinpuffer: 15,01 g Glycin in 11 Wasser lösen
8 M NaOH-Konzentrat: 32 g NaOH in 100 ml Wasser lösen
0,1 M Azetatpuffer: 5,76 ml Essigsäure glacial (100%) zu 11 Wasser zugeben, darin 29,22 g (0,5 M) NaCl lösen und das 8 M NaOH-Konzentrat bis pH 4,0 zutropfen (mindestens 50 Tropfen)
Wasser: Osmotisch gereinigt (auch für Operationen tauglich; Leitfähigkeit < 0,2µS/cm)

### A.) Cyano-aktivierte Sepharose 4B:

1) 1,2g Cyano-aktivierte Sepharose 4B in 240 ml 1 M HCl (kalt; 83,3 g in 11) 30 min aufschwellen. Diese in Chromatographie-Säule transferieren und mit 60 ml Wasser spülen. Danach 60 ml Coupling Puffer über die Chromatographie-Säule äquilibrieren. Nach der Umpufferung wird das Gel in 15 ml Couplingpuffer in zwei 7,5 ml-Röhrchen transferiert und 2 min bei 3000 rpm zentrifugiert.
   Der Überstand wird abgehoben auf 4,5 ml.
2.) Die Gele sind nun aktiviert für die Bindung von jeweils:
   1 ml Couplingpuffer mit 2 mg Galactosidase bzw.
   1 ml Couplingpuffer mit 5U Neuraminidase
   Jeweils einen Stoff zu den beiden 4,5 ml Gelen zugeben und 2 h bei 26°C schwenken.
3.) Blocken der restlichen aktivierten CN-Gruppen der Sepharose mit Glycin:
   Beide Gele mit den Enzymen werden in eine Chromatographiesäule überführt und mit 5 x 5 ml Coupling Puffer von nicht gebundenen Enzymen befreit. (Auch möglich mittels Zentrifugation: 6000 U/min, Abheben des Puffers vom Gel und Waschen mit Couplingpuffer und nochmaliger Zentrifugation; insgesamt 5 Mal)
   Zum Schluss:
      In 2 x 5 ml Couplingpuffer in 15 ml-Röhrchen transferieren und 2 min bei 3000 U/min zentrifugieren.
      Dann je 10 ml 0,2 M Glycin pH 8.0 zugeben und 20 Stunden bei 4-6°C stehen lassen; mehrmals schwenken.
4.) Reinigung des enzymaktivierten Gels von nicht gebundenem Glycin:
   Die Röhrchen werden zentrifugiert und der Überstand wird abgehoben.
   Die Reinigung des Enzym-Gels erfolgt durch 5-malige Zugabe von Coupling-Puffer und Zentrifugation bei 3000 U/min, 2 min und Abheben des Überstandes.
   Danach das gleiche Procedere mit 5-maliger Zugabe des Azetatpuffers statt des Coupling-Puffers.
   Die Lagerung erfolgt nach fünfmaligem Waschen mit 5 ml 1 M NaCl-Lösung, der 0,05 % Natriumazid zugesetzt wurde.
   Zum Ende wird das Gel in ein steriles Röhrchen transferiert und bei 4-6°C gelagert (= ready to use)

### B.) Cyano-aktivierte Magnetic Beads (CN-MB):

1.) 1,2 g CN-MB in 100 ml 1 M HCl (kalt; 83,3 g in 11) mehrmals waschen (mittels Zentrifugation und Magnet-Bindung). Danach mit Wasser spülen und mit Coupling Puffer äquilibrieren.
   Nach der Umpufferung werden die 1,2 g CN-MB in zwei 0,5 ml-Röhrchen transferiert und 2 min bei 3000 U/min zentrifugiert. Der Überstand wird abgehoben.
2.) Die Magnetic Beads sind nun aktiviert für die Bindung von jeweils:
   1 ml Couplingpuffer mit 2 mg Galactosidase
   1 ml Couplingpuffer mit 5U Neuraminidase
   Jeweils einen Stoff zu den je 0,6 g CN-MB zugeben und 2 h bei 26°C schwenken.
3.) Blocken der restlichen aktivierten CN-Gruppen der Magnetic Beads mit Glycin:
   Beide CN-MB-Enzyme werden mittels Magneten zurückgehalten und der Überstand abgehoben. Zugabe von 1 ml Coupling Puffer, schütteln und wiederum magnetische Bindung des CN-MG-Enzyms und Abheben des Überstandes. 4 Mal wiederholen. Dadurch werden nicht gebundene Enzyme entfernt.
   Dann je 1 ml 0,2 M Glycin pH 8,0 zugeben und 20 Stunden bei 4-6°C stehen lassen; mehrmals schwenken.
4.) Reinigung der enzymaktivierten Magnetic Beads von nicht gebundenem Glycin:
   Prinzip Magnetbindung und Abheben der Reinigungspuffer
   5-malige Zugabe von je 1 ml Coupling-Puffer, danach das gleiche Procedere mit 5-maliger Zugabe von 1 ml des Azetatpuffers.
   Die Lagerung von Galactosidase- und Sialidase-MBs erfolgt nach fünfmaligem Waschen mit 1 ml 1 M NaCl-Lösung, der 0,05 % Natriumazid zugesetzt wurde, in 0,5 ml solch einer Lösung bei 4-6°C (= ready to use)

Spaltung von Galactose bzw. Sialinsäure (Neuraminsäure) mit mittels Galaktosidase und Sialidase (bzw. Neuraminidase) aktivierter CN-Sephareose 4B oder CN-aktivierten Magnetic Beads.

### Verwendete Puffer:

0,1 M Phosphatpuffer pH 6.0 und 7.0
1 mg Gc-Proteine werden in 1 ml 0,1 M Phosphatpuffer pH 6,0 aufgelöst.
400 µg davon werden in 250 ml Wasser bei Raumtemperatur dialysiert (Dialyseschlauch; Filtrationsröhrchen); Dauer: 24h mit 3 x Wechsel.

### A.) Neuraminidase-Spaltung:

Das gereinigte Gc-Protein wird in 10 ml-Röhrchen mit der immobilisierten Neuraminidase (Sialidase), wie oben hergestellt, in Kontakt gebracht, entweder mit 3 ml aktivierter Sepharose oder mit 0.5 ml aktivierten Magnetic Beads und bei 37°C, 500 U/min, 2h inkubiert.

Nach der Inkubation
a.) Für Neuraminidase aktiviertes Sepharose-Gel: Zentrifugieren bei 6000 U/min für 2 min, danach 900 µl abheben.
   Das Sialidase-Gel mit 1 ml 0,1 M Phosphatpuffer pH 7,0 mischen (= einmal nachwaschen) und wiederum 2 min bei 6000 U/min zentrifugieren und wieder 900 µl abheben. Noch zwei weitere Male nachwaschen, zentrifugieren und abheben. Die abgehobenen Überstände vereinigen.
b.) Für Neuraminidase aktivierte Magnetic beads: Die Magnetic beads zurückhalten und 900 µl abheben.

Zu den MB 1 ml 0,1 M Phosphatpuffer pH 7,0 zugeben, mischen und wieder 900 µl abheben. Vorgang ein drittes Mal durchführen und die abgehobenen Überstände vereinigen.

Reinigungsschritte für Spaltprodukte aus a) und b): über einen Filter (10000 Da-Filter) bei 6000 U/min 5 min zentrifugieren, um die abgespaltenen Zuckerreste zu entfernen.

Den Überstand in 4 ml 0,1 M Phosphatpuffer pH 7,0 aufnehmen und wieder filtrieren. Dies noch ein drittes Mal durchführen. Den Überstand in 1 ml 0,1 M Phosphatpuffer pH 7,0 aufnehmen.

### B.) Die Spaltung mit Galaktosidase erfolgt analog zu der Spaltung mit Sialidase bzw. Neuraminidase über aktivierte Sepharose bzw. Magnetic Beads.

### Isolierung der gespaltenen Produkte. Umpufferung. Reinigung

### a) für Sepharose:

Den Filterrückstand mit 1 ml 0,9 %iger NaCl-Lösung aufschwemmen. Danach 2 min bei 6000 U/min zentrifugieren und 1 ml abheben.

Rest mit 1 ml 0,9 %iger NaCl-Lösung aufschwemmen, wieder 2 min bei 6000 U/min zentrifugieren und 1 ml abheben. Dies noch zwei Mal wiederholen.

### b) für Magnetic beads:

Den Filterrückstand mit 1 ml 0,9 %iger NaCl-Lösung aufschwemmen. Danach einfach mit einem Magnet die Beads zurückhalten und das Protein (=Alpha-Globulin-N-acetyl-glucosamin) abheben. Danach je 1 ml 0,9 %ige NaCl-Lösung zugeben, mixen, Magnet anlegen und den Überstand abheben. Vorgang 3 Mal wiederholen.

Alle Fraktionen zusammen in 10000 Da-Filter 8 min bei 6000 U/min zentrifugieren, um die Lösung einzuengen. (Alternativ kann eine Druckdialyse eingesetzt werden.)

Dem Rückstand 4 ml 0,9 %ige NaCl-Lösung zugeben und wieder 10 min bei 6000 U/min filtrieren. Der Rückstand ist etwa 500 µl.

Wiederum 4 ml 0,9 %ige NaCl-Lösung zugeben und nochmals 10 min bei 6000 U/min filtrieren; den Rückstand in 1 ml 0,9 %iger NaCl-Lösung aufnehmen.

Ergebnis: Sialinsäure abgetrennt; Galactose abgetrennt; keine DNA, RNA.

Schließlich erfolgt eine Filtrierung über ein 100.000 Da-Filter mittels Zentrifugation. GcMAF befindet sich im Filtrat (es hat rund 53 kDa), mögliche Enzyme des immobilisierten Materials sind abgetrennt im Filterrückstand. Damit ist eine Höchstreinigung des Produktes gegeben von über 99,5%!

Danach wird Cholecalciferol zugesetzt, um den Komplex GcMAF-Chlolecalciferol zu bilden. Der sich ergebende Komplex wird von überschüssigem Cholecalciferol durch ein 10000 Da-Molekularfilter getrennt (drei Mal 10 min mit 6000 U/min zentrifugieren und mit 10 mM PBS pH 7,4 waschen). Die Proteinbestimmung ergab 398 ng/ml des gewünschten Komplexes.

Es ist aber ebenso möglich, Cholecalciferol zu Beginn mit dem Gc-Protein reagieren zu lassen und danach die Zuckerreste wie beschrieben abzuspalten.

Für die folgenden Vergleichsversuche wurde dasselbe Herstellungsverfahren mit Calcitriol anstelle von Cholecalciferol durchgeführt. Es ergab sich dabei 440 ng/ml des Komplexes.

Die Bestimmung der Aktivierung der Phagozytose erfolgte wie beschrieben von Hammarstrom S and Kabat EA in "Studies on specificity and binding properties of the blood group A reactive hemagglutinin from Helix pomatia", Biochemistry 10: 1684-1692, 1971:
Peritoneale Maus-Zellen wurden in einer 24-Well-Platte auf Deckgläser geschichtet. Nach drei Stunden der Arzneimittelbehandlung wurden die Kulturen auf phagozytische Aktivität getestet. Rote Blutkörperchen vom Schaf (SRBC) wurden durch hämolytisches Kaninchenserum (Anti-Schaf-Rote Blutzellen C12HSB, Serotec Ltd. England) opsonisiert. Opsonisierte SRBC (0,5%) in RPMI 1640 (serumfrei) wurden auf jedem Makrophagen-beschichteten Deckglas überlagert und für 90 Minuten kultiviert. Die nicht internalisierten Erythrozyten wurden durch Eintauchen des Deckglases in eine hypotonische Lösung (1/5 PBS) lysiert. Die Makrophagen wurden mit Methanol fixiert, luftgetrocknet und mit Giemza-Färbung gefärbt. Die Anzahl der phagozytierten Erythrozyten pro Zelle wurde mikroskopisch bestimmt; 250 Makrophagen wurden für jeden Datenpunkt gezählt. Die Daten werden als Phagocytose-Index ausgedrückt, der als Prozentsatz von Makrophagen mit aufgenommenen Erythrozyten, multipliziert mit der durchschnittlichen Anzahl von Erythrozyten pro Makrophagen, definiert ist.

Es zeigt sich folgendes Ergebnis:

### [Tabellen0001]

**Tabelle 1**

| Substanz | Macrophagenaktivität |
|---|---|
| GcMAF-Calciol | 83,5 |
| GcMAF-Calcitriol | 73,2 |
| GcMAF, hergestellt wie beschrieben | 73,0 |
| GcMAF, gekauft von Metavectrum, Deutschland | 68 |

Die höhere Aktivität des selbst hergestellten GcMAF liegt vermutlich an der besseren Reinigung, insbesondere an der abschließenden Filtrierung mit dem 100.000 Da-Filter.

Die Bestimmung der Bildung von Superoxid-Radikal-Anionen in Monozyten aus humanem Vollblut wurde wie folgt durchgeführt:
Die Isolierung peripherer mononuklearer Zellen erfolgt mittels Dichtegradienten-Zentrifugation aus heparinisiertem Vollblut. Das Blut wird mit PBS (ohne Ca/Mg) 1:2 verdünnt und auf 15 ml Histopaque-1077 aufgeschichtet. Danach wird die Zellsuspension 30 min bei 700 g und 20°C zentrifugiert. Die Interphase mit den mononuklearen Zellen wird vorsichtig mit einer Pasteurpipette abgenommen, mit 30 ml PBS (ohne Ca/Mg) aufgefüllt und 5 min bei 350 g zentrifugiert. Der Überstand wird dekantiert, die Zellen werden in RPMI 1640 Medium aufgenommen und im Durchflusszytometer ausgezählt. Die Zellen werden auf 10⁵ Monozyten/ml eingestellt. Für den Testansatz wird in jedes 5 ml-Röhrchen 1 ml Zellsuspension pipettiert. Als Kontrolle wird LPS 100 ng/ml verwendet. Die Testsubstanz (GcMAF, GcMAF-Calciol bzw. GcMAF-Calcitriol) wird in einer Konzentration von 50 pg/ml eingesetzt. Die Zellen werden insgesamt 3 h im CO₂-Brutschrank inkubiert. 15 min vor Ende der Inkubation werden je 5 µl CD45-V450 Antikörper und 1 µl einer 1 mM MitoSOX-stock-Lösung dazugegeben. Danach wird die Zellsuspension mit je 2 ml RPMI-Medium aufgefüllt und 5 min bei 350 g bei Raumtemperatur zentrifugiert. Der Überstand wird dekantiert und die Zellen werden in 500 µl RPMI-Medium resuspendiert. Die Zellen werden im Durchflusszytometer gemessen und analysiert. Die Auswertung erfolgt über die mittlere Fluoreszenzintensität, wie beschrieben von Saharuddin Bin Mohamad, Hideko Nagasa Wa, Yoshihiro Uto and Hitoshi Hori in "Preparation of Gc Protein-derived Macrophage Activating Factor (GcMAF) and its Structural Characterization and Biological Activities", Anticancer Research 22: 4297-4300 (2002):

### Assay zur Bildung von Superoxid

Die Methode wurde ausgehend von der von Johnston et al. beschriebenen modifiziert. Kurz gesagt, nach der dreistündigen Probenbehandlung wurden die Platten zweimal mit PBS (-)und einmal mit Krebs-Ringer-Phosphatpuffer, pH 7,4, gewaschen, und 1,5 ml von 50 µM Cytochrom C in Krebs-Ringer-Phosphatpuffer wurde zugegeben, und Phorbolmyristatacetat (PMA) wurde hinzugefügt zu einer endgültigen Konzentration von 5 µg/ml in jedem Well und für 90 Minuten in einem befeuchteten Inkubator kultiviert.

Die Reaktion wurde mittels eines Eisbades gestoppt. Das kultivierte Medium wurde in ein Eppendorf-Röhrchen gegeben und bei 8000 g zentrifugiert. Die optische Dichte des Überstandes wurde spektrometrisch bei 550 nm mit Referenz bei 540 nm (U-2000, Hitachi) unter Verwendung von Gemischen von Platten ohne Zellen als Leermessung bestimmt. Die Konzentration von reduziertem Cytochrom C wurde unter Verwendung der Gleichung E₅₅₀ₙₘ = 2,1.10⁻¹ M⁻¹cm⁻¹ bestimmt.

Es zeigt sich folgendes Ergebnis:

### [Tabellen0002]

**Tabelle 2**

| Substanz | Superoxidaktivität |
|---|---|
| GcMAF-Calciol | 142 |
| GcMAF-Calcitriol | 188 |
| GcMAF, hergestellt wie beschrieben | 199 |
| GcMAF, gekauft von Metavectrum, Deutschland | 150 |

Man sieht, dass die Superoxidaktivität des erfindungsgemäßen Komplexes am geringsten von allen getesteten Substanzen und vergleichbar zu reinem GcMAF ist. Es kann also durch die Bindung von Calciol an GcMAF dessen Makrophagenaktivität signifikant gesteigert werden, ohne dass dies durch den Nachteil einer Erhöhung der Superoxidaktivität erkauft werden muss.

Der erfindungsgemäße Komplex kann oral verabreicht werden. Es wurde der Vitamin D-Gehalt im Blut nach oraler Gabe gemessen, und dieser stieg von 35,9 nM (vor der Verabreichung) auf 44,25 nM (12 Stunden nach Verabreichung) an. Der erfindungsgemäße Komplex wird also bei oraler Verabreichung vom Körper aufgenommen. Der erfindungsgemäße Komplex ist außerdem nicht toxisch.

Es konnte auch ein signifikanter Anstieg monozytärer Zellen, die sich in der Folge in Makrophagen umwandeln, nach oraler Verabreichung des erfindungsgemäßen Komplexes gezeigt werden.

## Patentansprüche

1. Dimerer Komplex aus selektiv deglycosyliertem Vitamin D bindenden Protein (GcMAF) und Cholecalciferol (Calciol).

2. Komplex nach Anspruch 1 als Arzneimittel.

3. Verfahren zur Herstellung eines Komplexes nach Anspruch 1, **dadurch gekennzeichnet, dass** man Vitamin D bindendes Protein mit Calciol reagieren lässt und davor oder danach selektiv deglycosyliert, wobei man schließlich das sich ergebende Produkt mit einem Molekularsieb mit einem Cut-Off-Wert von mehr als 60 kDa von Verunreinigungen größeren Molekulargewichts, insbesondere von Enzymen der selektiven Deglycosylierung, trennt.

4. Verfahren nach Anspruch 3, **dadurch gekennzeichnet, dass** man das sich ergebende Produkt mit einem Molekularsieb mit einem Cut-Off-Wert von höchstens 10 kDa von abgespaltenen Zuckerresten trennt.

## Claims

1. A dimeric complex of a selectively deglycosylated vitamin D-binding protein (GcMAF) and cholecalciferol (calciol).

2. The complex as claimed in claim 1 as a drug.

3. A method for producing a complex as claimed in claim 1, **characterized in that** a vitamin D-binding protein is reacted with calciol and selectively deglycosylated before or after being reacted, wherein, using a molecular sieve with a cutoff value of more than 60 kDa, the resulting product is finally separated from impurities with a larger molecular weight, in particular enzymes of the selective deglycosylation process.

4. The method as claimed in claim 3, **characterized in that** the resulting product is separated from cleaved sugar residues using a molecular sieve with a cutoff value of at most 10 kDa.

## Revendications

1. Complexe dimère constitué de la protéine de liaison à la vitamine D sous forme déglycosylée (GcMAF) et de cholécalciférol (calciol).

2. Complexe selon la revendication 1, servant de médicament.

3. Procédé de fabrication d'un complexe selon la revendication 1, **caractérisé en ce que** l'on fait réagir la protéine de liaison à la vitamine D avec du calciol, la première subissant une déglycosylation sélective en amont ou en aval de cette réaction, pour ensuite séparer le produit obtenu, à l'aide d'un tamis moléculaire dont la valeur d'exclusion est supérieure à 60 kDa, d'impuretés dépassant ce poids moléculaire, notamment d'enzymes destinées à la déglycosylation sélective.

4. Procédé selon la revendication 3, **caractérisé en que** l'on sépare le produit obtenu, à l'aide d'un tamis moléculaire dont la valeur d'exclusion est inférieure ou égale à 10 kDa, de résidus de sucre tels qu'ils sont présents suite au clivage.
